# EUROPEAN PATENT APPLICATION

(11) **EP 1 894 989 A1**
(43) Date of publication of application: **05.03.2008**
(21) Application number: 06120005.1
(22) Date of filing: 01.09.2006
(51) Int. Cl.: C11D 1/62, C11D 17/00, C11D 17/04, C11D 1/38

(54) **Pasty Composition for Sanitary Ware**

(71) Applicant: The Procter and Gamble Company, Cincinnati, Ohio 45202 (US)
(72) Inventor: Evers, Marc François Theophile, 1853 Strombeek-Bever (BE); Garmyn, Wim Peter Agnes, 2018 Antwerpen (BE); Guarracino, Mario, 64029 Silvi Marina (Teramo) (IT); Scialla, Stefano, 00128 Rome (IT)
(74) Representative: Kellenberger, Jakob

(57) **Abstract**

The present invention is directed to pasty compositions suitable for cleaning and/or deodorizing sanitary ware, wherein the compositions comprise a cationic surfactant.

## Description

### Technical field

The present invention relates to pasty compositions for cleaning and/or deodorizing sanitary ware such as toilet bowls, urinals, and sinks. More specifically, the pasty compositions of the present invention provide excellent cleaning and/or deodorizing benefit as well as excellent durability profile.

### Background of the invention

Compositions for cleaning and/or deodorizing sanitary ware such as toilets or urinals have long been sought by consumers, in both residential and commercial environments. In an attempt to meet the demand for sanitary cleaning compositions or deodorizers, numerous products have been developed and are presently available in the martketplace. In general, existing products are sold as solids, liquids or gel to provide the desired effect. In the context of toilet bowls, it is known to use sanitary agents in toilet hang-in baskets, which are attached through a holder to the toilet bowl edge, whereby the sanitary agent is released with each flushing action. Such sanitary agents are known in the form of solid blocks and recently also in gel form.
In spite of the long-lasting benefit provided by such products, the main disadvantage or inconvenience felt by the consumers occurs while replacing or re-filling by hand known sanitary agent for subsequent use. Those manipulations may be perceived as highly unhygienic and time-consuming. Also, the presence of known hanging devices baskets may disturb intermediary cleaning activities.
Other well known sanitary cleaning products are available in liquid form and packaged in squeezable bottles. Such liquid cleaning products however do only perform their cleaning action for a limited period.

Partial solutions to these drawbacks have been provided with for example in US-B1-6,667,286 and EP-B1-1086199 which disclose adhesive sanitary agents for direct application to a sanitary object and which are claimed to be washed away only after a large number of rinsing actions. However, the disclosed agents make use of specific adhesion promoters which may undesirably increase formulation complexity. Also, the mineral build-up prevention properties and the adhesion/dissolution profiles of the disclosed products may still be improved.

It is therefore an objective of the present invention to provide a composition which is capable of providing excellent cleaning and/or deodorizing performance to sanitary ware, while providing excellent mineral build-up prevention benefits and improved adhesion/dissolution profiles.

Advantageously, the composition according to the present invention is capable of providing long-term and sustained cleaning and/or air freshening benefits. Also the method of the present invention is able to provide such benefits in a highly controlled manner as the user is allowed to choose the specific amount of composition which needs to be applied to the sanitary ware.

A further advantage associated with the composition according to the present invention is that the user is not required to be in contact with unhygienic sanitary surface or objects. It is still a further advantage that the composition according to the present invention is formulated from very simple and inexpensive material, and may be easily employed in both residential and commercial establishments.

Other advantages and more specific properties of the method according to the present invention will be clear after reading the following description of the invention.

### Summary of the invention

The present invention relates to a pasty composition suitable for direct application to the surface of a sanitary ware, wherein the composition comprises a cationic surfactant and is suitable for cleaning and/or deodorizing the sanitary ware.
In another embodiment, the present invention is directed to a method for imparting durable cleaning and/or deodorizing properties to sanitary ware, the method comprising the step of directly applying a composition as above-described directly to the sanitary ware.

The present invention further encompasses the use of a composition as above-described, for cleaning and/or deodorizing sanitary ware.

### Detailed description of the invention

### PASTY COMPOSITION

The composition according to the present invention is in the form of a pasty composition. By "pasty" composition, it is meant to encompass any of paste, gel, or cream composition. By "sanitary ware", it is meant herein any of toilet bowl, urinal, bathtub or sink. In the context of the present invention, the sanitary ware is preferably selected from toilet bowl and urinal, more preferably from toilet bowl.

The viscosity measurements mentioned in the present application have all been obtained with a TA Instruments/Advanced rheometer AR 1000, plate-plate system with stainless steel flat plate having a diameter of 40 mm, at a temperature of 20°C with a gap setting of 1000 microns, and at a shear rate of 25 s⁻¹.

Accordingly, the viscosity of the pasty composition of the present invention is preferably at least 5.000 mPas when measured with a TA Instruments/Advanced rheometer AR 1000 at 20°C. Typically, the viscosity of the composition according to the invention is comprised between 5.000 mPas and 500.000 mPas, preferably between 10000 mPas and 200.000 mPas, more preferably between 15.000 mPas and 150.000mPas, and most preferably between 25.000 mPas and 100.000 mPas.

A pasty composition having a viscosity of at least 5.000 mPas, when measured with an AR 1000 Rheometer at 20°C, is believed to ensure better surface coverage of the applied composition to the treated surface. Also, viscous compositions, as herein described, are allowed to act on the surface more intimately than non-viscous liquid compositions and typically result in better cleaning and/or deodorizing performances.

The compositions of the present invention are advantageously provided with adhesiveness properties. The compositions herein are indeed capable of strongly and instantaneously adhering to the treated sanitary ware surface. The obtained adhesion shall typically be sufficient to prevent such pasty composition to be entirely detached under pressure exercised by the water flush. In contrast, the composition according to the present invention should only progressively be detached from the treated surface. Typically, the pasty composition shall be integrally washed away only after a large number of rinsing actions. Generally, the composition as described herein shall only be integrally rinsed-off after at least 30, preferably after at least 50, more preferably after at least 70, most preferably after between 80 and 200 rinse cycles.

### CATIONIC SURFACTANT

According to the present invention, the pasty composition for use herein comprises a cationic surfactant. Suitable cationic surfactants for use according to the present invention may be selected from any cationic surfactant commonly known in the art.

Suitable cationic surfactants to be used herein include derivatives of quaternary ammonium, phosphonium, imidazolium, sulfonium compounds, and mixtures thereof. Preferred cationic surfactants for use herein are derivatives of quaternary ammonium. Accordingly, suitable cationic surfactants for use herein include, but are not limited to quaternary ammonium compounds wherein one or two of the hydrocarbon groups linked to nitrogen are a saturated or partially unsaturated, linear or branched alkyl group of 6 to 30 carbon atoms, preferably of 10 to 25 carbon atoms, and more preferably of 12 to 20 carbon atoms, and wherein the other hydrocarbon groups (i.e. three when one hydrocarbon group is a long chain hydrocarbon group as mentioned hereinbefore or two when two hydrocarbon groups are long chain hydrocarbon groups as mentioned hereinbefore) linked to the nitrogen are independently substituted or unsubstituted, linear or branched, alkyl chain of from 1 to 4 carbon atoms, preferably of from 1 to 3 carbon atoms, and more preferably are methyl groups.

Other suitable cationic surfactants for use in the compositions of the present invention include trimethyl quaternary ammonium compounds like myristyl trimethylsulfate, cetyl trimethylsulfate and/or tallow trimethylsulfate, and mixtures thereof. Such trimethyl quaternary ammonium compounds are commercially available from Hoechst, or from Albright & Wilson under the trade name EMPIGEN CM®. Other cationic surfactants useful herein are also described in U.S. Patent 4,228,044, Cambre, issued October 14, 1980.

Particularly preferred cationic surfactants for use herein are selected from the group consisting of diethyl ester hydroxyethyl methyl ammonium methyl (DEEHMAMS), wherein the hydrocarbon groups linked to the carbonyls are preferably a saturated, linear or branched alkyl group of 16 to 18 carbon atoms; diethyl ester dimethyl ammonium chloride (DEEDMAC), wherein the hydrocarbon groups linked to the carbonyls are preferably a saturated, linear or branched alkyl group of 16 to 18 carbon atoms, preferably an alkyl group of 18 carbon atoms; triethanol amine ester methyl ammonium methyl sulphate (TEEMAMS), wherein the hydrocarbon groups linked to the carbonyls are preferably a saturated or unsaturated, linear or branched alkyl group of 16 to 18 carbon atoms, more preferably an unsaturated, linear alkyl group of 17 carbon atoms; hydroxyethyl dimethyl ammonium chloride, wherein the hydrocarbon group linked to nitrogen is preferably a saturated, linear or branched alkyl group of 12 to 14 carbon atoms; hydroxyethyl dimethyl ammonium chloride, wherein the hydrocarbon group linked to nitrogen is preferably a saturated, linear or branched alkyl group of 8 to 10 carbon atoms; lauryl methyl bis hydroxyethyl ammonium chloride; dimethyl benzalkonium chloride, wherein the hydrocarbon group linked to nitrogen is preferably a saturated or unsaturated, linear or branched alkyl group of 12 to 18 carbon atoms, and mixtures thereof.
More preferably, the cationic surfactant is selected from the group consisting of DEEHMAMS, DEEDMAC, TEEMAMS, dimethyl benzalkonium chloride, and mixtures thereof.

In the context of the present invention, it has been surprisingly discovered that cationic surfactants when included in pasty compositions herein provide excellent adhesion and dissolution profile of said compositions applied to the treated sanitary ware surface. More surprisingly, the Applicant has further discovered that pasty compositions described herein and incorporating cationic surfactants provide improved resistance to flushing actions over time when applied to the treated surface, in comparison to pasty compositions comprising e.g. anionic and/or amphoteric surfactants. Without wishing to be bound by theory, it is believed that cationic surfactants when incorporated into the corresponding pasty compositions described herein ensure better interaction with the generally negatively charged surface of the treated sanitary ware.

Preferably, the composition according to the present invention provides adequate adhesion without requiring additional use of adhesion promoters or tackifiers.

### OPTIONAL INGREDIENTS IN THE COMPOSITIONS HEREIN

### Acidic compound

As an optional, but highly preferred ingredient, the pasty composition for use herein further comprises an acidic compound. According to the present invention, the pasty composition may typically comprise at least 5%, preferably at least 10%, more preferably at least 15% by weight of said composition of said acidic compound.

Suitable acidic compounds for use herein may be selected from any commonly known organic or inorganic acidic compounds, and mixtures thereof. Suitable organic acidic compounds for use herein include but are not limited to citric acid, maleic acid, adipic acid, oxalic acid, malic acid, succinic acid, tartaric acid, salicylic acid, aspartic acid, glutaric acid, malonic acid, and mixtures thereof. As a way of example, suitable inorganic acidic compounds comprise but are not limited to sulphamic acid, phosphoric acid, nitric acid, sulphonic acid, sulphuric acid, hydrochloric acid, or their salts or mixtures thereof.

Other acidic compounds useful herein include commonly known polymeric acids. Suitable polymeric acids for use herein comprise, but are not limited to polyacrylic polymers or acrylic-maleic copolymers, which are available e.g. from BASF under the tradenames Sokalan® CP5 or CP7 or CP9. Also useful herein are polyacrylic phosphono end group polymers or acrylic-maleic phosphono end group copolymers (available e.g. from Rohm & Haas under the tradenames Acusol® 420 or 470 or 425); and polyacrylic sulphono end group polymers or acrylic-maleic sulphono end group copolymers.

Preferably, acidic compound for use in the present invention is selected from acidic compounds in solid form. In an even more preferred embodiment, the acidic compound is in granular/powder form. Preferred acidic compounds for use herein include but are not limited to citric acid, maleic acid, and sulphamic acid. In a very preferred execution, the composition of the present invention comprises citric acid.

Without wanting to be bound by theory, the Applicant has found that acidic compound for use in the present invention contributes in providing improved inorganic build-up prevention benefits in spite of the highly viscous properties of the corresponding pasty composition. In particular, it has been discovered that the pasty composition of the present invention provides e.g. excellent limescale and hydroxyapatite prevention benefits. It is believed that the acidic compounds for use herein act as builders helping at keeping calcium and magnesium cations soluble in water, and therefore preventing the formation of the corresponding mineral deposits.

Additionally, and while still not being bound by theory, it is believed that acidic compounds in granular form may promote the formation of suitable network within the pasty composition of the present invention. As a result, the acidic compound is believed to advantageously participate in obtaining suitable viscosity and adhesion properties. Advantageously, the acidic compound for use herein provides an economically cheaper alternative to commonly known adhesion or viscosity promoters.

### Tackifying system

The composition according to the present invention may optionally comprise a tackifier.
Suitable tackifiers for use herein may be selected from any tackifier commonly known in the art, the choice of which is well within the capability of those skilled in the art. Suitable tackifiers for use in the compositions of the present invention may typically be defined as long or long-chained organic molecules, which are at least partly hydrophilic, and wherein the hydrophilic part of the tackifier interacts at least in part with water molecules and becomes tacky.
As a way of example suitable tackifiers for use herein include but are not limited to polyethylene glycol, polywax, cellulose, particularly sodium carboxymethyl cellulose, hydroxyethyl cellulose, hydroxypropyl cellulose, or polysaccharides such as xanthan gum, agar agar, gellan gum, acacia gum, carob bean flour, or guar gum or starch. It is also possible to use polymers such as polyacrylates, polyvinyl alcohols, polyvinyl pyrrolidones, and mixtures thereof. Suitable tackifiers may also include alginates, diurethanes, gelatins, pectines, oleyl amines, alkyl dimethyl amine oxides, or alkyl ether sulfates.
Preferred tackifiers are selected from the group consisting of short chain alcohols, glycols, polyglycols, glycerines, and mixtures thereof. More preferably, the tackifier is a short chain alcohol such as ethanol or isopropanol.

### Additional surfactants

As another preferred optional ingredient, the composition described herein may comprise additional surfactants. The presence of said surfactants in the compositions herein may allow providing compositions with desired viscosity by appropriately chosen surfactants and levels thereof.

Virtually any surfactant known in the art may be used as additional surfactant. Suitable surfactants may be advantageously selected from the group consisting of anionic, nonionic, cationic, amphoteric, zwiterrionic surfactants, and mixtures thereof. It is preferred to use anionic surfactant in the composition according to the present invention.

Accordingly, the compositions according to the present invention comprise up to 50%, preferably of from 0.1% to 20%, more preferably of from 1% to 10%, and most preferably of from 1% to 5% by weight of the total composition of said additional surfactant, or mixtures thereof.

Suitable nonionic surfactants to be used herein are alkoxylated fatty alcohol nonionic surfactants that can be readily made by condensation processes that are well known in the art. Indeed, a great variety of such alkoxylated fatty alcohols are commercially available which have very different HLB values. Preferred nonionic surfactants for use in the compositions according to the invention are the condensation products of ethylene oxide with alcohols having a straight alkyl chain, having from 6 to 22 carbon atoms, wherein the degree of ethoxylation is from 1 to 15, preferably from 5 to 12. Such suitable nonionic surfactants are commercially available from Shell, for instance, under the trade name Dobanol® or from Shell under the trade name Lutensol®.

Suitable amphoteric surfactants to be used in the compositions according to the present invention include amine oxides having the following formula R₁R₂R₃NO wherein each of R1, R2 and R3 is independently a saturated substituted or unsubstituted, linear or branched alkyl groups of from 1 to 30 carbon atoms, preferably of from 6 to 30 carbon atoms, more preferably of from 10 to 20 carbon atoms, and most preferably of from 8 to 18 carbon atoms. Preferred amine oxides for use herein are for instance natural blend C₈-C₁₀ amine oxides as well as C₁₂-C₁₆ amine oxides commercially available from Hoechst.

Suitable anionic surfactants herein include water soluble salts or acids of the formula ROSO₃M wherein R is preferably a C₁₀-C₂₄ hydrocarbyl, preferably an alkyl or hydroxyalkyl having a C₁₀-C₂₀ alkyl component, more preferably a C₁₂-C₁₈ alkyl or hydroxyalkyl, and M is H or a cation, e.g., an alkali metal cation (e.g., sodium, potassium, lithium), or ammonium or substituted ammonium (e.g., methyl-, dimethyl-, and trimethyl ammonium cations and quaternary ammonium cations, such as tetramethylammonium and dimethyl piperdinium cations and quaternary ammonium cations derived from alkylamines such as ethylamine, diethylamine, triethylamine, and mixtures thereof). Preferred anionic surfactants for use in the compositions herein are the alkyl benzene sulfonates, alkyl sulfates, alkyl alkoxylated sulfates, and mixtures thereof.

### Perfumes

The compositions according to the present invention may comprise, as an optional ingredient, perfume ingredient selected from the group consisting of a cyclic terpene/sesquiterpene perfume, such as eucalyptol, cedrol, pinocarveolus, sesquiterpenic globulul alcohol; linalo; tetrahydrolinalo; verdox (cyclohexadiyl 2 tetryl butyl acetate); 6,3 hexanol; and citronellol and mixtures thereof.

The compositions according to the present invention may typically comprise from 0.01 % to 10%, preferably from 0.01% to 5%, more preferably from 0.01% to 1 %, and most preferably from 0.01% to 0.1% by weight of the total composition of said perfume ingredient.

### Solvents

As another optional ingredient, the composition herein may comprise one or more solvents or mixtures thereof. Solvents for use herein include all those known in the art for use in hard-surface cleaner compositions. Suitable solvents can be selected from the group consisting of: aliphatic alcohols, ethers and diethers having from about 4 to about 14 carbon atoms, preferably from about 6 to about 12 carbon atoms, and more preferably from about 8 to about 10 carbon atoms; glycols or alkoxylated glycols; glycol ethers; alkoxylated aromatic alcohols; aromatic alcohols; terpenes; and mixtures thereof. Aliphatic alcohols and glycol ether solvents are most preferred, particularly those with vapour pressure of about 0.05 mm Hg at 25°C and 1 atmosphere pressure (about 6.66 Pa).

Aliphatic alcohols, of the formula R-OH wherein R is a linear or branched, saturated or unsaturated alkyl group of from about 1 to about 20 carbon atoms, preferably from about 2 to about 15 and more preferably from about 5 to about 12, are suitable solvents. Suitable aliphatic alcohols are methanol, ethanol, propanol, isopropanol or mixtures thereof. Among aliphatic alcohols, ethanol and isopropanol are most preferred because of their high vapour pressure and tendency to leave no residue.

Suitable glycols to be used herein are according to the formula HO-CR1R2-OH wherein R1 and R2 are independently H or a C2-C10 saturated or unsaturated aliphatic hydrocarbon chain and/or cyclic. Suitable glycols to be used herein are dodecaneglycol and/or propanediol.

Particularly preferred glycol ethers have a terminal C3-C6 hydrocarbon attached to from one to three ethylene glycol or propylene glycol moieties to provide the appropriate degree of hydrophobicity and, preferably, surface activity. Examples of commercially available solvents based on ethylene glycol chemistry include mono-ethylene glycol n-hexyl ether (Hexyl Cellosolve®) available from Dow Chemical. Examples of commercially available solvents based on propylene glycol chemistry include the di-, and tri-propylene glycol derivatives of propyl and butyl alcohol, which are available from Arco under the trade names Arcosolv® and Dowanol®.

### Chelating agents

Another class of optional compounds for use herein includes chelating agents. Chelating agents may be incorporated in the compositions herein in amounts ranging up to 10.0%, preferably 0.01 % to 5.0% by weight of the total composition.

Suitable phosphonate chelating agents to be used herein may include alkali metal ethane 1-hydroxy diphosphonates (HEDP), alkylene poly (alkylene phosphonate), as well as amino phosphonate compounds, including amino aminotri(methylene phosphonic acid) (ATMP), nitrilo trimethylene phosphonates (NTP), ethylene diamine tetra methylene phosphonates, and diethylene triamine penta methylene phosphonates (DTPMP). The phosphonate compounds may be present either in their acid form or as salts of different cations on some or all of their acid functionalities. Preferred phosphonate chelating agents to be used herein are diethylene triamine penta methylene phosphonate (DTPMP) and ethane 1-hydroxy diphosphonate (HEDP). Such phosphonate chelating agents are commercially available from Monsanto under the trade name DEQUEST®.

Polyfunctionally-substituted aromatic chelating agents may also be useful in the compositions herein. See U.S. patent 3,812,044, issued May 21, 1974, to Connor et al. Preferred compounds of this type in acid form are dihydroxydisulfobenzenes such as 1,2-dihydroxy -3,5-disulfobenzene.

A preferred biodegradable chelating agent for use herein is ethylene diamine N,N'-disuccinic acid, or alkali metal, or alkaline earth, ammonium or substitutes ammonium salts thereof or mixtures thereof. Ethylenediamine N,N'-disuccinic acids, especially the (S,S) isomer, have been extensively described in US patent 4, 704, 233, November 3, 1987, to Hartman and Perkins. Ethylenediamine N,N'-disuccinic acid is, for instance, commercially available under the tradename ssEDDS® from Palmer Research Laboratories.

Suitable amino carboxylates to be used herein include ethylene diamine tetra acetates, diethylene triamine pentaacetates, diethylene triamine pentaacetate (DTPA), N-hydroxyethylethylenediamine triacetates, nitrilotri-acetates, ethylenediamine tetrapropionates, triethylenetetraaminehexa-acetates, ethanol-diglycines, propylene diamine tetracetic acid (PDTA) and methyl glycine di-acetic acid (MGDA), both in their acid form, or in their alkali metal, ammonium, and substituted ammonium salt forms. Particularly suitable amino carboxylates to be used herein are diethylene triamine penta acetic acid, propylene diamine tetracetic acid (PDTA) which is, for instance, commercially available from BASF under the trade name Trilon FS® or Trilon M® and methyl glycine di-acetic acid (MGDA).

Further carboxylate chelating agents to be used herein include salicylic acid, aspartic acid, glutamic acid, glycine, malonic acid or mixtures thereof.

In addition to the above-mentioned optional ingredients, the composition according to the present invention may also further comprise other components selected from the group of fragrances, thickeners, colorants, dyes, optical brighteners, builders, chelants, preservatives, solvents, buffering agents, radical scavengers, stabilizers, dye scavengers, adhesion promoters, rheology modifiers, bleaching systems, and mixtures thereof.

### METHOD FOR IMPARTING CLEANING AND/OR DEODORIZING PROPERTIES

In another embodiment of the present invention, it is provided a method for imparting durable cleaning and/or deodorizing properties to sanitary ware, the method comprising the step of directly applying a composition as above-described directly to the sanitary ware.

By "durable", it is meant herein that the cleaning and/or deodorizing benefit provided by the composition of the present invention, when applied to a sanitary ware, typically lasts for up to at least 30, preferably for at least 50, more preferably for at least 70, most preferably for up to between 80 and 200 rinse cycles.

Due to the particular viscosity/adhesiveness profiles of the composition of the present invention, it is possible to apply it directly to the sanitary ware surface. Typically, the pasty composition of the invention may be applied with any suitable device capable of distributing viscous compositions. Suitable devices will be easily recognized by those skilled in the art of dispensing devices. Those include, but are not limited to, squeezable tubes, cartridge, syringe-type devices, pasty glue dispenser-type devices.

The method for imparting durable cleaning and/or deodorizing properties according to the invention, prevents the user from contacting by hand dirtied and unhygienic surfaces or objects, via using suitable dispensing devices. The method according to the invention also gives the user a simple and highly controlled way of applying the composition as the latter is allowed to choose the specific amount of composition which needs to be applied to the sanitary ware. Also, the method of the invention provides the user with the flexibility to choose exactly the location(s) of the sanitary ware where the composition needs to be applied. This provides the consumer a totally new, hygienic and exciting toilet care experience.

Incidentally, and according to another embodiment of the present invention, it is provided a method of imparting durable mineral build-up prevention benefit to sanitary ware, the method comprising the step of directly applying a composition as above-described directly to the sanitary ware.

Compositions according to one embodiment of the invention are preferably applied to the treated sanitary ware surface by using suitable dispensing devices. Preferably, compositions described herein are applied through flexible pouches or tubes. Suitable dispensing devices will be easily recognized by those skilled in the art of dispensing device for pasty compositions.

### USE OF THE COMPOSITION FOR CLEANING AND/OR DEODORIZING

The present invention is further directed to the use of a composition as described herein, for cleaning and/or deodorizing sanitary ware. In another embodiment of the invention, it is provided the use of a composition as described herein, for preventing mineral build-up on sanitary ware.

### EXAMPLES

These following compositions were made comprising the listed ingredients in the listed proportions (weight %). The examples herein are met to exemplify the present invention but are not necessarily used to limit or otherwise define the scope of the present invention. Compositions I to VIII are compositions according to the present invention, whereas composition IX and X are comparative examples.

| Ingredients: (% by weight) | I | II | III | IV | V |
|---|---|---|---|---|---|
| DEEHMAMS | 7 | 7.5 | 8.3 | - | 15 |
| Uniquat® | 13 | 22.5 | 16.7 | 13 | 15 |
| TEEMAMS | - | - | - | - | - |
| DEEDMAC | - | - | - | 7 | - |
| CMBHAC | - | - | - | - | - |
| Citric S40 | 30 | 18 | 25 | 30 | 18 |
| Sodiumsulphate | 45 | 50 | 43 | 46 | 43 |
| Perfume | 2 | 2 | 2 | 2 | 2 |
| PEG 9000MW | - | - | 5 | - | - |
| PVP | - | - | - | - | 2 |
| Betaine | - | - | - | - | - |
| LAS | - | - | - | - | - |
| Amineoxide | - | - | - | - | - |
| NaCS | - | - | - | - | - |
| Hexylcellosolve | - | - | - | - | - |
| Glycerol | 2 | - | - | - | - |
| Waters & Minors | Up to 100 | | | | |

| Ingredients: (% by weight) | VI | VII | VIII | IX | X |
|---|---|---|---|---|---|
| DEEHMAMS | 7.5 | - | - | - | - |
| Uniquat® | 22.5 | 16.7 | - | - | - |
| TEEMAMS | - | 8.3 | 7.5 | - | - |
| DEEDMAC | - | - | - | - | - |
| CMBHAC | - | - | 22.5 | - | - |
| Citric S40 | 18 | 39 | 29 | 23 | 32.5 |
| Sodiumsulphate | 45 | 28 | 28 | - | 20 |
| Perfume | 2 | 4 | 4 | 2 | 4 |
| PEG 9000MW | - | 2 | 5 | - | 1 |
| PVP | - | - | 1 | - | - |
| Betaine | - | - | - | 32 | - |
| LAS | - | - | - | - | 32.5 |
| Amineoxide | 3 | - | - | - | 3 |
| NaCS | - | - | - | 23 | - |
| Hexylcellosolve | - | - | - | 12 | - |
| Glycerol | - | - | - | - | - |
| Waters & Minors | Up to 100 | | | | |

| | | | | | |
|---|---|---|---|---|---|
| DEEHMAMS is a Diethyl Ester Hydroxyethyl Methyl Ammonium Methyl Sulphate, supplied by Stepan under the tradename Stepantex VK90®. TEEMAMS is a Triethanol Amine Ester Methyl Ammonium Methyl Sulphate, supplied by Degussa under the tradename Rewoquat V3620®. DEEDMAC is Diethyl Ester Dimethyl Ammonium Chloride, supplied by Degussa under the tradename Rewoquat V3282®. Uniquat® is a C12-C18 Benzalkonium chloride, supplied by Lonza. Citric acid S40 is supplied by Jungbunzlauer. CMBHAC is Coconut methyl bishydroxyethyl ammoniumchloride, supplied by AKZO-Nobel. PEG is polyethylene glycol, supplied by BASF. PVP is polyvinyl pyrrolidone, supplied by BASF. Betaine is Lauryl amido propyl betaine supplied by Mc Intyre. LAS is linear alkylbenzene sulphonate, supplied by Tensachem. Amine oxide is A0728®, supplied by Tomah. NaCS is sodium cumenesulphonate, purchased from Rutgers Organics. Hexylcellosolve is supplied by Dow Chemicals. Glycerol is supplied by P&G Chemicals. | | | | | |

The pH of these examples is below 7. Compositions I to VIII have a viscosity of at least 5.000 mPas, when measured with a TA Instruments/Advanced rheometer AR 1000 at a temperature of 20°C with a gap setting of 1000 microns, and at a shear rate of 25 s⁻¹. Compositions I to VIII exhibit excellent adhesion profile to regular toilet bowl and limescale build-up prevention performance, as well as outstanding cleaning performance.

### COMPARATIVE DATA

A comparative dissolution profile performance study is conducted according to the following test method. Porcelain white tiles (typically 17 cm X 11 cm) are weighed using an analytical balance with an accuracy of 0.01 gr. A pasty composition sample is then applied without applying any force to the surface of the tile via a syringe, forming a 9 cm long paste strip having a section plane of 15mm X 2mm. Again, the weight of the treated tile is recorded. The tiles are then held on vertical position under a tap, and for a period of 10 seconds, 1 litre of tap water having a hardness of 15 gpg is poured over the tiles. The flushed tiles are then left to dry for 15 minutes. After repeating this flush cycle 10 times, the weight of the flushed tiles are measured and the percentage of product removed from the corresponding tiles is measured.
The ability of the compositions to remain on the treated surface is measured through the percentage of product removed from the corresponding tiles. The lower the percentage of removed product, the higher the ability of the corresponding composition to remain on the treated surface.

This experiment, which is aimed at determining resistance of different compositions to flushing actions over time, involved two compositions according to the present invention (Compositions II and III as above-described), and two comparative compositions not according to the present invention (Compositions IX and X). For all four compositions, the percentage of product removed from the treated surface after 10 flushing actions is measured.

| | Composition II | Composition III | Composition IX | Composition X |
|---|---|---|---|---|
| % of product removed | 25 | 25 | 70 | 38 |

The above results clearly show the improved resistance to flushing actions performance obtained with compositions according to the present invention (e.g. compositions II and III), i.e. compositions comprising a cationic surfactant, versus comparative compositions comprising anionic and/or amphoteric surfactants.

The dimensions and values disclosed herein are not to be understood as being strictly limited to the exact numerical values cited. Instead, unless otherwise specified, each such dimension is intended to mean both the recited value and a functionally equivalent range surrounding that value. For example, a dimension disclosed as "40 mm" is intended to mean "about 40 mm".

## Claims

1. A pasty composition suitable for direct application to the surface of a sanitary ware, wherein said composition comprises a cationic surfactant and is suitable for cleaning and/or deodorizing said sanitary ware.

2. A composition according to claim 1 wherein the viscosity of said composition is at least 5.000 mPas, when measured with a TA Instruments/Advanced rheometer AR 1000 at a temperature of 20°C with a gap setting of 1000 microns, and at a shear rate of 25 s⁻¹.

3. A composition according to any of the preceding claims, wherein said cationic surfactant is selected from the group consisting of DEEHMAMS, DEEDMAC, TEEMAMS, dimethyl benzalkonium chloride, and mixtures thereof.

4. A composition according to any of the preceding claims, wherein said composition further comprises an acidic compound, preferably selected from the group consisting of citric acid, maleic acid, sulphamic acid, and mixtures thereof.

5. A composition according to any of the preceding claims, wherein said composition further comprises a tackifier, preferably selected from the group of polyethylene glycol, polywax, cellulose, polysaccharides, polyacrylates, polyvinyl alcohols, polyvinyl pyrrolidones, alginates, diurethanes, gelatins, pectines, oleyl amines, alkyl dimethyl amine oxides, alkyl ether sulfates, ethanol, isopropanol, glycols, polyglycols , glycerines, and mixtures thereof.

6. A composition according to any of the preceding claims, wherein the viscosity of said composition is comprised between 5.000 mPas and 500.000 mPas, preferably between 10000 mPas and 200.000 mPas, more preferably between 15.000 mPas and 150.000mPas, and most preferably between 25.000 mPas and 100.000 mPas, when measured with a TA Instruments/Advanced rheometer AR 1000 at a temperature of 20°C with a gap setting of 1000 microns, and at a shear rate of 25 s⁻¹.

7. A composition according to any of the preceding claims, which further comprises components selected from the group consisting of fragrances, thickeners, colorants, dyes, optical brighteners, builders, chelants, preservatives, solvents, buffering agents, radical scavengers, stabilizers, dye scavengers, adhesion promoters, bleaching systems, and mixtures thereof.

8. A composition according to any of the preceding claims in a form selected from of a gel, a paste, and a cream.

9. A composition according to any of the preceding claims, wherein said sanitary ware is a toilet bowl.

10. A method for imparting durable cleaning and/or deodorizing properties to sanitary ware, said method comprising the step of applying a composition according to any of the preceding claims directly to said sanitary ware.

11. A method according to claim 10, whereby said composition is washed away only after at least 30, preferably after at least 50, more preferably after at least 70, most preferably after between 80 and 200 rinse cycles.

12. The use of a composition according to any of claims 1 to 9, for cleaning and/or deodorizing sanitary ware.
